# EUROPÄISCHE PATENTSCHRIFT

(11) **EP 1 420 836 B1**
(45) Veröffentlichungstag und Bekanntmachung des Hinweises auf die Patenterteilung: **14.10.2009**
(21) Anmeldenummer: 02754093.9
(22) Anmeldetag: 23.08.2002
(51) Int. Cl.: A61M 1/00

(54) **PORTSYSTEM FÜR EINEN PERKUTAN IMPLANTIERTEN PORT**
PORT SYSTEM FOR A PERCUTANEOUSLY IMPLANTED PORT
SYSTEME D'ACCES POUR ACCES A IMPLANTATION PERCUTANEE

(30) Priorität: 31.08.2001 DE 10142637; 06.09.2001 DE 20114795 U; 29.10.2001 DE 10153341
(43) Veröffentlichungstag der Anmeldung: 26.05.2004
(73) Patentinhaber: F. Hoffmann-La Roche AG, 4070 Basel (CH); Roche Diagnostics GmbH, 68305 Mannheim (DE)
(72) Erfinder: DENOTH, Patrik, CH-4912 Aarwangen (CH); NIEDERHÄUSER, Sandro, CH-4914 Roggwil (CH); VALIQUER, Astrid, CH-1029 Villars-Ste-Croix (CH)
(74) Vertreter: Wess, Wolfgang
(86) Internationale Anmeldenummer: PCT/CH2002/000462
(87) Internationale Veröffentlichungsnummer: WO 2003/020343

(56) Entgegenhaltungen:
- WO-A-90/11103
- WO-A-99/34754
- US-A- 4 488 877
- US-A- 4 645 494
- US-A- 5 810 792

## Beschreibung

Die Erfindung betrifft ein Portsystem, das einen perkutan implantierbaren Port und eine Konnektiervorrichtung als Komponenten enthält. Besonders bevorzugt findet die Erfindung auf den Gebieten der Infusion, Dialyse, Perfusion und bei Messzwecken dienenden Anwendungen Verwendung.

In der Medizinaltechnik, insbesondere in der Humanmedizin, müssen Fluidfiihrungssysteme, die beispielsweise Katheter umfassen oder durch Katheter alleine gebildet werden, miteinander dicht und steril verbunden werden. Besonders kritisch ist die Verbindung im Falle von Körperzugangsvorrichtungen, sogenannten Ports, die dauerhaft in einem biologischen Gewebe, insbesondere im menschlichen Körper, implantiert sind. Seit längerem sind subkutan, d. h. vollständig implantierbare Ports im Einsatz, die für langfristige oder intermittierende Medikamentenapplikationen eingesetzt werden. Komplikationen der subkutan implantierten Ports sind Infektionen, Katheterokklusionen und -migrationen. Außerdem erlauben die betreffenden Portsysteme nur unter sehr schwierigen Umständen eine kontinuierliche Medikamentenabgabe, wie sie beispielsweise bei der kontinuierlichen Abgabe von Morphinen in der Behandlung von chronischen Schmerzen notwendig ist.

Aus der WO 99/34754 ist eine Übertragungsvorrichtung zum Übertragen von beispielsweise Daten oder einem Medikament bekannt, mit einem Verankerungsteil, das subkutan an einem Knochen befestigt werden kann und einer Verbindungsvorrichtung, die lösbar mit der Übertragungsvorrichtung verbunden ist. Die Verbindungsvorrichtung weist einen Kanal mit einer Membran auf. Durch diesen Kanal kann beispielsweise mittels einer Injektionsnadel die die Membran durchsticht, ein Medikament über einen im Verankerungsteil gebildeten oder in das Verankerungsteil eingesetzten Kanal in einen Knochen oder ein Blutgefäß geleitet werden. Bei Abgabe eines Medikaments aus der Injektionsnadel in einen Hohlraum vor dem Kanal oder direkt in den Kanal kommt es zu Verwirbelungen in dem Medikament, da der Durchmesser der Injektionsnadel sowohl kleiner ist als der Durchmesser des Hohlraums, als auch der Durchmesser des Kanals. Aus der US 4,488,877 ist ein perkutanes Implantat für eine peritoneale Dialyse bekannt, an das ein flexibler Katheder angeschlossen und von dem ein flexible Katheder abgenommen werden kann, ohne dass es einer Operation bedarf. Der Katheder wird dabei mit dem starren Implantat an einem flexiblen Hülsenteil verbunden und durch einen Clip zusammengehalten. Dabei kann der Katheder durch das perkutane Implantat hindurchreichen oder in einer inneren Kavität des Implantats enden. Endet der Katheder im Implantat, weist das Kathederende einen kleineren Durchmesser auf als der das Ende aufnehmende Teil des Transplantats, wodurch es im Übergangsbereich zu Turbulenzen der Dialyseflüssigkeit kommt. Die US 4,645,494 beschreibt ein peritoneales Vorrichtungssystem mit einem starren rohförmigen Implantat, einer Anschlussstabilisierungshilfe und einer Anschlussgarnitur für die Dialyse. Zur Abgabe der Dialyseflüssigkeit wird die Anschlussgarnitur durch eine Öffnung in die Anschlussstabilisierungshilfe eingerührt und fest mit dieser verbunden. Dadurch wird ein Ventilmechanismus in dem Implantat geöffnet, so dass Flüssigkeit in den Körper einfließen oder aus dem Körper ausfließen kann. Im verbundenen Zustand ist das Ende der Anschlussgarnitur in einen Kanal im Implantat eingeführt. Dies führt zu einer abrupten Änderung des Durchmessers der Flüssigkeit führenden Kanäle und damit zu Turbulenzen in der Flüssigkeit im Bereich dieses Übergangs. Aus der WO 90/11103 sind zahlreiche Stuckverbindungen eines Verbindungselements mit einem Port bekannt. Dabei ist der Strömungsquerschnitt der Verbindungskanüle immer kleiner als der des Anschlussbereichs des Ports.

Eine wesentliche Innovation stellt in diesem Zusammenhang das von der Anmelderin entwickelte Portsystem DiaPort® dar. DasDiaPort®-System ist für die kontinuierliche intraperitoneale und intraumbilikal-venöse Insulinadministration vorgesehen. Im Gegensatz zu den subkutanen Systemen wird der Port des DiaPort®-Systems nicht vollständig, sondern perkutan implantiert. Ein kleiner Teil des Ports ragt über die Hautoberfläche nach außen hervor und ist von außen, d. h. extern, sichtbar und für einen Anschluss an ein externes Fluidführungssystem zugänglich. Das DiaPort®-System wird beispielsweise in der EP 0 867 196, der EP 0 867 197 und der EP 0 867 198 beschrieben.

Das externe Fluidführungssystem des DiaPort®-Systems weist an einem Ende eine Verbindungskanüle auf, die an ihrem vorderen freien Ende kugelförmig verdickt ist.

Wenn das externe Fluidfiihrungssystem an das implantierte Fluidführungssystem angeschlossen ist, d. h. im konnektierten Zustand, ragt die Verbindungskanüle in ein Gehäuse des Ports ein und durch eine in dem Portgehäuse aufgenommene, dichtende Membran hindurch. Das kugelförmig verdickte Ende der Verbindungskanüle verhindert eine Beschädigung der Membran durch die eindringende Verbindungskanüle. Da die Membran aus einem elastischen Material besteht, das sich hinter dem verdickten Kanülenende elastisch und dicht an die Verbindungskanüle anlegt, bildet die kugelförmige Verdickung gleichzeitig auch eine Konnektiervorrichtung, die das externe Fluidführungssystem an dem Port, nämlich an dessen Membran, befestigt.

Es ist eine Aufgabe der Erfindung, die mechanische Verbindung zwischen einem perkutan implantierten oder implantierbaren Port und einem externen Fluidführungssystem zu verbessern. Die Verbindung soll auf einfache Weise herstellbar und wie- der lösbar sein und eine stabile Konnektierung der Teile gewährleisten.

Ein Portsystem, wie die Erfindung es betrifft, umfasst ein implantierbare erstes Fluidführungssystem, ein externes zweites Führungssystem, einen perkutan implantierbaren Port und eine Konnektiervorrichtung. In einem implantierten Zustand verbindet der perkutane Port die beiden Fluidführungssysteme fluidisch und mechanisch. Ein Gehäuse des Ports, das im implantierten Zustand mit einer Oberseite aus dem Gewebe herausragt, bildet ein erstes Konnektierelement. Die Konnektiervorrichtung bildet ein zweites Konnektierelement. Das zweite Fluidführungssystem weist an einem
Ende einen Konnektierkopf auf, der das zweite Fluidführungssystem in einem Befestigungseingriff, in dem sich die Konnektierelemente im konnektierten Zustand miteinander befinden, an dem Portgehäuse befestigt. Der Befestigungseingriff der Konnektierelemente ist lösbar, vorzugsweise ist der Befestigungseingriff wiederholt herstellbar und lösbar.

Das Portsystem kann im Rahmen beispielsweise einer venösen Behandlung angewendet werden, beispielsweise in der Onkologie, der Behandlung von Aids, Schmerz, Mukoviszidose, Blutgerinnungsdefizienzen, Asthma, Spastizität, Knochenmarksentzündung, zur parenteralen Ernährung, in der Diabetestherapie zur Insulinverabreichung und vieles mehr.

Nach der Erfindung ist der Befestigungseingriff form- und kraftschlüssig. Die Befestigung ist derart, dass die Konnektierelemente und dadurch das Portgehäuse und der Konnektierkopf mit einer Presskraft gegeneinander gepresst werden. Da der Konnektierkopf durch den Befestigungseingriff an dem Portgehäuse und nicht an einer in dem Gehäuse aufgenommenen Membran gehalten wird, ist die erfindungsgemäße Konnektierung sehr stabil. Zwischen der das Portgehäuse nach außen steril abdichtenden Membran und einem die Membran durchragenden Verbindungselement, vorzugsweise eine Verbindungskanüle, des Konnektierkopfs wirken keine oder allenfalls vernachlässigbare mechanischen Kräfte. Soweit äußere Kräfte überhaupt wirken, werden sie von dem Portgehäuse aufgenommen. Aufgrund der Entlastung von äußeren Kräften kann ferner sichergestellt werden, dass die Verbindungskanüle des Konnektierkopfs in dem Portgehäuse eine stets definierte Stellung einnimmt und die Fluidströmung an einem Übergang zwischen den beiden Fluidführungssystemen nicht durch äußere Kräfte gestört wird. Erfindungsgemäß weist der Konnektierkopf eine Verbindungskanüle auf, und die Verbindungskanüle und ein in das Portgehäuse gefüllter Anschlussbereich des ersten Fluidsystems weisen den gleichen Strömungsquerschnitt auf, um Turbulenzen in dem Fluid zu vermeiden.

Die Konnektierelemente sind vorzugsweise so ausgebildet, dass der Befestigungseingriff hergestellt wird, indem der Konnektierkopf gegen das Portgehäuse gedrückt wird. Eine Achse, entlang der dieses Drücken stattfindet, wird im Folgenden auch als Konnektierachse bezeichnet. Sie fällt vorzugsweise mit einer Längsachse einer Verbindungskanüle zusammen, die von dem Konnektierkopf abragt. Bevorzugterweise wird der . Konnektierkopf durch den Befestigungseingriff in Bezug auf die Konnektierachse axial unbeweglich mit dem Portgehäuse verbunden.

Der Befestigungseingriff ist vorzugsweise ein Rasteingriff Eines der Konnektierelemente bildet einen Rastvorsprung und das andere eine Rastnase, die den Rastvorsprung im konnektierten Zustand hintergreift. Bei dem Rastvorgang gleitet die Rastnase über den Rastvorsprung und wird vorzugsweise bereits allein aufgrund der Gleitbewegung quer zu der Richtung der Gleitbewegung entgegen einer rückstellenden Elastizitätskraft bewegt. Die rückstellende Elastizitätskraft bewirkt, dass die Rastnase sich im Verlauf der weiteren Gleitbewegung wieder quer zu der Richtung der Gleitbewegung bis gegen eine hinter dem Rastvorsprung gebildete Rastschulter vorbewegt. Die Richtung der Gleitbewegung weist vorzugsweise senkrecht zu einer Oberseite des Portgehäuses, d. h. sie weist vorzugsweise auch senkrecht zur Hautoberfläche, wenn der Port implantiert ist. Auf diese Weise werden die bei der Konnektierung wirkenden Kräfte quer zur Hautoberfläche minimal gehalten.

In bevorzugter Ausführung ist der Konnektierkopf im Befestigungseingriff der Konnektierelemente relativ zu dem Portgehäuse um eine senkrecht zu der Oberseite des Portgehäuses weisende Achse, die vorzugsweise mit der Konnektierachse zusammenfällt, drehbar. Im implantierten Zustand des Ports ist der Konnektierkopf relativ zu dem Portgehäuse somit um eine zur Hautoberfläche senkrechte Achse drehbar, so dass auf den Konnektierkopf tangential zur Hautoberfläche wirkende Kräfte, verursacht beispielsweise durch Körperbewegungen, nicht in das Portgehäuse eingeleitet werden. In dem bevorzugten Rasteingriff gleitet die Rastnase bei einer Drehbewegung an der von dem Rastvorsprung gebildeten Rastschulter entlang. Die Drehachse fällt vorzugsweise mit der Längsachse der Verbindungskanüle zusammen.

In besonders bevorzugter Ausführung bildet die Konnektiervorrichtung eine Zange.

Das zweite Konnektierelement bildet eine Backe dieser Zange und wird im Folgenden daher auch als Konnektierbacke bezeichnet. Die Konnektiervorrichtung weist einen Grundkörper auf, von dem die Konnektierbacke elastisch abspreizbar ist, um den Befestigungseingriff herzustellen und wieder zu lösen. Obgleich der Grundkörper selbst eine Gegenbacke der Zange bilden kann, umfasst die Konnektiervorrichtung bevorzugter zusätzlich zu dem zweiten Konnektierelement und dem Grundkörper eine weitere Konnektierbacke, die ebenfalls elastisch von dem Grundkörper abspreizbar ist. Die in diesem Falle zwei Konnektierbacken weisen vorzugsweise die gleiche Form auf und sind symmetrisch zu einer Symmetrieachse, die sich quer zu der Konnektierachse erstreckt.

Die wenigstens eine Konnektierbacke ist über eine Spreizachse hinaus verlängert, um ein Greifelement zu bilden. Als Spreizachse wird die Achse bezeichnet, um die die Konnektierbacke ihre Spreizbewegung ausführt. Die weitere Konnektierbacke, falls eine solche vorgesehen ist, ist über ihre Spreizachse hinaus in gleicher Weise zur Ausbildung eines Greifelements verlängert. Das wenigstens eine Greifelemente, vorzugsweise die beiden Greifelemente, dient oder dienen dem Lösen des Befestigungseingriffs der Konnektierelemente. Während nämlich die Herstellung des Befestigungseingriffs vorzugsweise automatisch dadurch erfolgt, dass der Konnektierkopf gegen die Oberseite des Portgehäuses gedrückt wird, kann das Lösen des Befestigungseingriffs durch Betätigung der Greifelemente, zumindest erleichtert werden. Vorzugsweise werden die Konnektierelemente durch eine Betätigung des wenigstens einen Greifelements so aus ihrem Befestigungseingriff gebracht, dass der Konnektierkopf ohne die Ausübung von Zugkräften von dem Portgehäuse gelöst und entfernt werden kann.

In einer Weiterbildung sind die vorzugsweise zwei Greifelemente, die zum Lösen der Konnektierung um die Spreizachse aufeinander zu drückbar sind, an ihren Enden aufeinander zu gebogen. In einer bevorzugten Ausführung bilden sie einen geschlossenen Bogen, so dass ein augenförmiges Greifteil entsteht. Die Ausbildung des Greifteils in Form eines Auges mit einer runden, in der Draufsicht beispielsweise ellipsenförmigen Außenkontur, hat den Vorteil, dass die Konnektiervorrichtung sich in keiner Weise in Kleidungsstücken verfangen kann, so dass noch sicherer ausgeschlossen werden kann, dass unerwünschte Kräfte über die Konnektiervorrichtung in das Portgehäuse eingeleitet werden können.

Die Spreizachse der wenigstens einen Konnektierbacke kann grundsätzlich zwar quer zu der Konnektierachse weisen, bevorzugter weist sie jedoch in die gleiche Richtung wie die Konnektierachse.

Die Konnektiervorrichtung wird vorzugsweise von einem einzigen Körper gebildet. Grundsätzlich könnte das zweite Konnektierelement jedoch als ein separates Teil gefertigt und bewegbar gegen ein Federelement an einem Grundkörper der Konnektiervorrichtung abgestützt sein. Falls die Konnektiervorrichtung mehrere, als separate Teile gefertigte zweite . Konnektierelemente umfasst, würde dies für jedes dieser Konnektiereleniente gelten.

Der Konnektierkopf und die Konnektiervorrichtung können je einstückig, aber separat voneinander gefertigt und anschließend miteinander verbunden sein. Bevorzugter wird jedoch der Konnektierkopf mit der Konnektiervorrichtung in einem Stück gefertigt.

Falls eine Zange die Konnektiervorrichtung bildet, kann solch eine Konnektiervorrichtung ein von dem Konnektierkopf separates Teil bilden. Der Konnektierkopf würde in eine für die Konnektierung geeignete Stellung relativ zu dem Portgehäuse gebracht, und anschließend würde die separate Konnektiervorrichtung so angelegt werden, dass der Konnektierkopf form- und kraftschlüssig mit dem Portgehäuse verbunden wird, vorzugsweise indem er an das Portgehäuse gepresst wird. Eine wesentlich einfachere Handhabung ergibt sich jedoch, wenn die Konnektiervorrichtung von vornherein mit dem Konnektierkopf verbunden ist, so dass diese beiden Teile nicht erst bei der Konnektierung des Konnektierkopfs und des Portgehäuses miteinander verbunden werden müssen. Vorzugsweise bildet der Konnektierkopf die Konnektiervorrichtung unmittelbar.

Weitere vorteilhafte Ausgestaltungen der Erfindung werden durch die Unteransprüche beschrieben.

Nachfolgend wird die Erfindung anhand eines Ausführungsbeispiels erläutert. An dem Ausführungsbeispiel offenbar werdende Merkmale bilden je einzeln und in jeder Merkmalskombination die Gegenstände der Ansprüche vorteilhaft weiter. Es zeigen:
- Figur 1: Komponenten eines Portsystems je in Einzeldarstellung,
- Figur 2: das Portsystem in einem konnektierten Zustand im Längsschnitt,
- Figur 3: den für eine Implantierung vorgesehenen Teil des Portsystems,
- Figur 4: ein Membrangehäuse des Portsystems,
- Figur 5: eine Schnittdarstellung eines Konnektierkopfs des Portsystems,
- Figur 6: eine Ansicht der Unterseite des Konnektierkopfs und
- Figur 7: eine Ansicht der Oberseite des Konnektierkopfs.

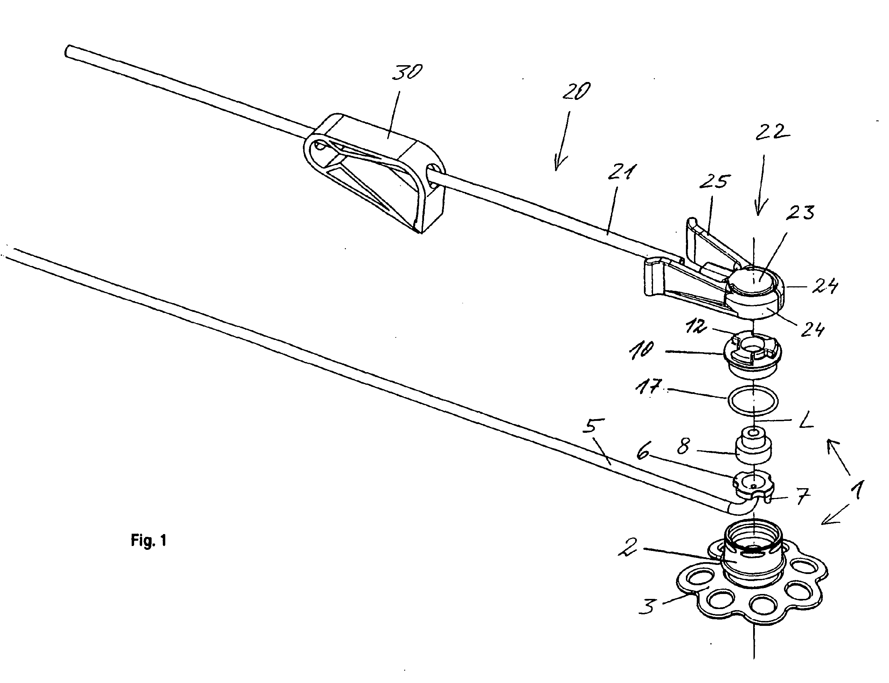

Figur 1 zeigt die Komponenten eines Portsystems. Das Portsystem umfasst ein implantierbares, erstes Fluidführungssystem, das im Ausführungsbeispiel von einem Katheter 5 und einem tellerförmigen Stützkörper 6 gebildet wird, einen Perkutanport und ein externes, zweites Fluidführungssystem 20. Der Perkutanport ist für eine perkutane Implantation vorgesehen und weist ein hohlzylindrisches Hauptgehäuse 2 und einen Verankerungskörper 3 auf. Der Verankerungskörper 3 ragt an einer Unterseite des Hauptgehäuses 2 radial und zu seinem äußeren Rand hin peripher abfallend von dem Hauptgehäuse 2 ab. Er dient der Verankerung des Perkutanports unter der Haut. Der Perkutanport umfasst ferner eine Membran 8, ein hülsenförmiges Membrangehäuse 10 und einen O-Ring 17. Zur Bildung des Perkutanports wird die Membran 8 in das Membrangehäuse 10 eingesetzt. Das Membrangehäuse 10 wird mit der eingesetzten Membran 8 in das Hauptgehäuse 2 eingesetzt und befestigt, beispielsweise mittels einer im Ausführungsbeispiel gewählten Schraubverbindung. Im verbundenen Zustand bilden das Hauptgehäuse 2 und das Membrangehäuse 10 gemeinsam das Portgehäuse. Vor dem Einsetzen des Membrangehäuses 10 wird der Katheter 5 durch eine Gehäuseöffnung an der Unterseite des Hauptgehäuses 2 hindurchgezogen, bis der Stützkörper 6 auf einem Boden des Hauptgehäuses 2 aufsitzt.

In Figur 1 sind die Komponenten des Perkutanports und der Stützkörper 6 entlang einer gemeinsamen Längsachse L dargestellt. Die Zentrizität in Bezug auf die gemeinsame Längsachse L bleibt im montierten Zustand erhalten. Die Längsachse L erstreckt sich insbesondere durch die Gehäuseöffnung im Boden des Hauptgehäuses 2, eine zentrische Öffnung im Stützkörper 6, einen Durchgang durch die Membran 8 und durch das Membrangehäuse 10.

Der Katheter 5 und der Stützkörper 6 werden in einer ersten Ausführungsform von einem bogenförmig vorgefertigten Anschlussstück miteinander verbunden. Der Katheter 5 wird aus einem biokompatiblen Kunststoff vorzugsweise endlos extrudiert und auf Länge abgeschnitten. Der Stützkörper 6 wird vorzugsweise zusammen mit dem gekrümmten Anschlussstück ebenfalls aus einem biokompatiblen Kunststoffmaterial im Spritzguss einstückig geformt. Der Katheter 5 wird anschließend mit dem freien Ende des gekrümmten Anschlussstücks gefügt und stoffschlüssig verbunden, vorzugsweise wird das Anschlussstück mit dem Stützkörper 6 an das Ende des Katheters 5 angespritzt. In einer zweiten Ausführungsform, in der der Katheter 5 beispielsweise aus PUR gebildet wird, wird der Katheter 5 endlos exdrudiert und auf Länge abgeschnitten. Der Stützkörper 6 wird wieder einstückig gespritzt ohne Anschlussstück, und bei dem Spritzvorgang an ein Ende des abgelängten Katheters angespritzt. Anschießend wird der Katheder 5 in seinem sich an den Stützkörper 6 anschließenden Abschnitt thermisch umgeformt, so dass im Anschluss an den Stützkörper 6 ein bogenförmiger Abschnitt erhalten wird.

Das externe Fluidführungssystem als dritte Hauptkomponente des Portsystems ist im Ganzen mit dem Bezugszeichen 20 bezeichnet. Es umfasst einen Katheter 21, der mit einem Ende an einen Konnektierkopf 22 angeschlossen ist. Der Konnektierkopf 22 dient der Herstellung der Fluidverbindung zwischen dem Katheter 21 und dem implantierbaren oder implantierten Katheter 5 und der Befestigung des externen Katheters 21 an dem Perkutanport. Für die Befestigung bildet der Konnektierkopf 22 eine Konnektiervorrichtung in der Form einer Spreizzange mit zwei von Zangenbacken gebildeten Konnektierelementen 24 und entsprechend zwei je von einem Zangenflügel gebildeten Greifelementen 25. Eine Spreizachse der Zange weist parallel zu der Längsachse L. Die Spreizachse erstreckt sich in einem Abstand zu der Längsachse L durch einen Bereich des Konnektierkopfs 22 auf der Höhe des Übergangs von den Konnektierelementen 24 zu ihrem jeweiligen Greifelement 25. Um die Fluidverbindung mit dem implantierbaren oder implantierten Fluidführungssystem 5, 6 und die mechanische Verbindung mit dem Port herzustellen, wird der Konnektierkopf 22 entlang der Längsachse L gegen die Oberseite des Ports gedrückt und hierdurch mit dem Port verrastet. Die Längsachse L bildet eine Konnektierachse, entlang der die Bewegung des Konnektierkopfs 22 relativ zu dem Port bei der Konnektierung stattfindet.

Auf den Katheter 21 ist ferner eine Quetschklammer 30 aufgeschoben. Die Ouetschklammer 30 bildet einen in Bezug auf den Katheter 21 festen, oberen Klammerschenkel und einen gegen den oberen Klammerschenkel anpressbaren unteren Klammerschenkel. Der untere Klammerschenkel wird durch einen lösbaren Rasteingriff in der Stellung gehalten, in der er gegen den oberen Klammerschenkel angepresst ist. Im Rasteingriff wird der Katheter 21 zwischen den beiden Klammerschenkeln so gequetscht, dass die Fluidzufuhr oder -abfuhr durch den Katheter 21 unterbrochen wird. In Figur 1 sind die beiden Klammerschenkel nicht gegeneinander gepresst.

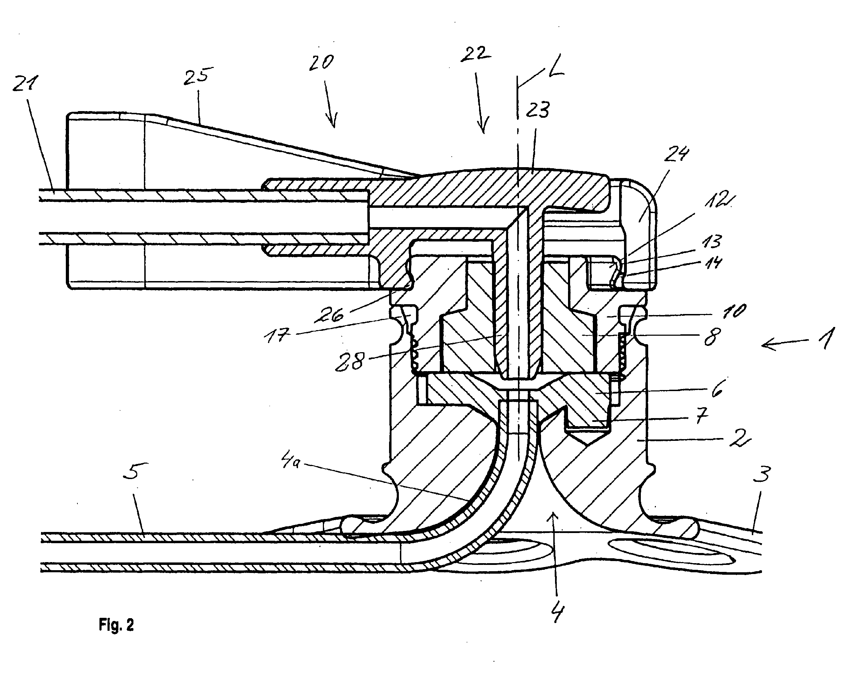

Figur 2 zeigt das Portsystem im konnektierten Zustand. Mit dem Bezugszeichen 1 ist der Perkutanport vom Verankerungskörper 3 bis zu dem Membrangehäuses 10 bezeichnet. Der Perkutanport 1 umfasst das Hauptgehäuse 2, den Verankerungskörper 3, das Membrangehäuse 10, die Membran 8 und den O-Ring 17. Der Stützkörper 6 wird wegen seiner festen Verbindung mit dem Katheter 5 im Ausführungsbeispiel zu dem implantierten oder implantierbaren ersten Fluidführungssystem gezählt. Wie aus den Figuren 1 und 2 erkennbar, bilden der Stützkörper 6 ein Zentrierelement 7 und das Hauptgehäuse 2 eine Aufnahme, in die das Zentrierelement 7 im montierten Zustand hineinragt, um den Stützkörper 6 und insbesondere dessen Durchgangsöffnung in Bezug auf die Längsachse L in dem Hauptgehäuse 2 zu platzieren, vorzugsweise wie im Ausführungsbeispiel um dem gekrümmten Anschlussstück eine Richtung vorzugeben. Das Zentrierelement 7 sichert den Stützkörper 6 gegen eine Drehbewegung um die Längsachse L. Der Stützkörper 6 weist an seinem Rand eine Mehrzahl von Aussparungen auf, die Durchgriffe für ein Werkzeug bilden, um für die Entfernung des implantierten Katheters 5 den Stützkörper 6 greifen und aus dem Hauptgehäuse 2 ziehen zu können. Eine einzige Aussparung würde grundsätzlich auch genügen.

Ein weiteres besonderes Merkmal des Ports 1 ist die Bildung einer stetig gerundeten Führung 4a für das Anschlussstück des implantierten Fluidführungssystems 5, 6. Die Führung 4a erstreckt sich von der Gehäuseöffnung im Boden des Hauptgehäuses 2 bis zu dem unteren, peripheren Rand des Hauptgehäuses 2 und dient der knickfreien Umlenkung des Katheters 5 bis in die Gehäuseöffnung. An der Unterseite des Hauptgehäuses 2 ist nicht nur die Führung 4a gebildet, sondern auch ein Öffnungstrichter 4, in dem das gekrümmte Anschlussstück des Katheters 5 vor Druckbelastungen weitestgehend geschützt ist. Der Öffnungstrichter 4 öffnet sich von der Gehäuseöffnung aus trompetenförmig, wie beispielhaft gezeigt.

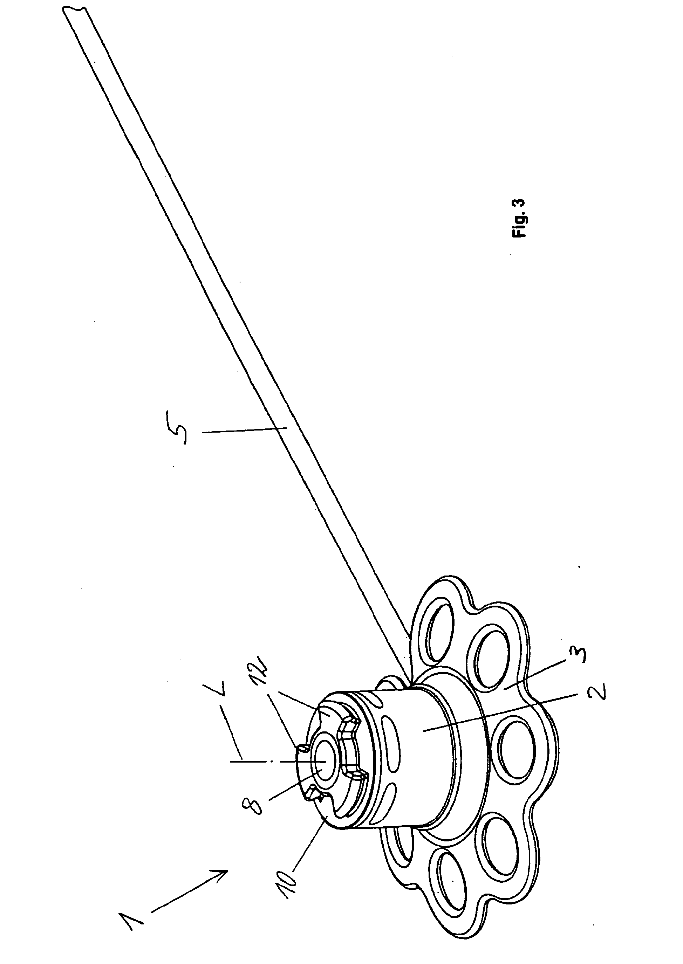

In Figur 3 ist der für die Implantierung vorgesehene Teil des Portsystems nochmals alleine in einer perspektivischen Ansicht dargestellt.

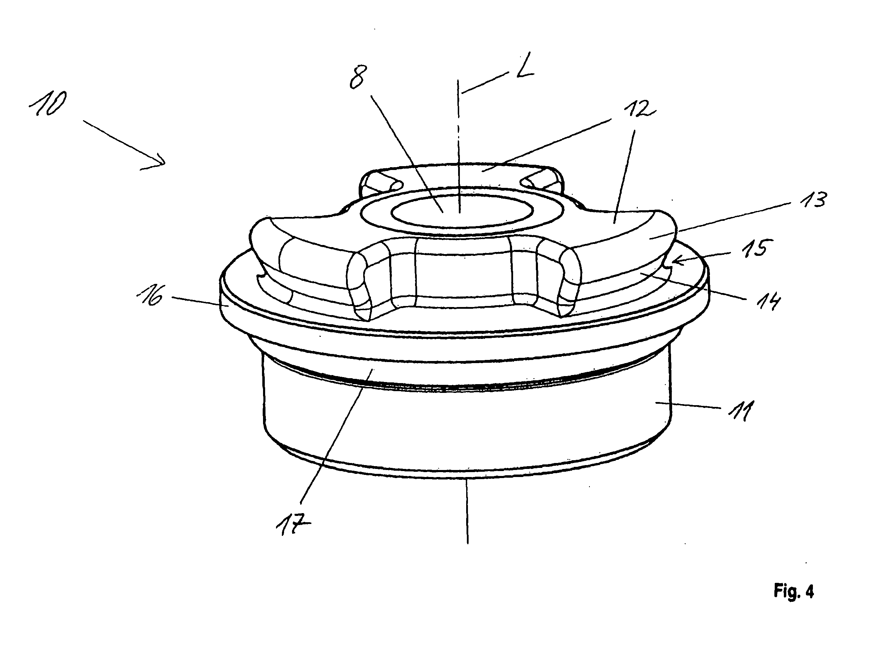

Figur 4 zeigt das Membrangehäuse 10 mit der eingesetzten Membran 8 und dem angelegten O-Ring 17 in einer perspektivischen Ansicht.

Die Membran 8 ist kreiszylindrisch mit einem oberen, kleineren Querschnitt und einem unteren, größeren Querschnitt. Durch die Membran 8 erstreckt sich ein zentraler Durchgang, der bereits durch die Materialelastizität der Membran steril verschlossen wird. Die Membran 8 wird im eingesetzten Zustand in dem Membrangehäuse 10 unter einer gewissen Radialspannung vorkomprimiert gehalten. Im montierten Zustand (Figuren 2 und 3) drückt die Unterseite des Membrangehäuses 10 gegen die Oberseite des Stützkörpers 6 und presst ihn in seinen Sitz in dem Hauptgehäuse 2. Gleichzeitig wird auch die Membran 8 mit ihrer Unterseite gegen den Stützkörper 6 gedrückt, so dass um die zentrale Durchgangsöffnung des Stützkörpers 6 umlaufend eine sterile Dichtung gebildet wird. Der Stützkörper 6 fällt von seinem Rand zu seiner zentralen. Durchgangsöffnung hin ein wenig ab. Die Durchgangsöffnung des Stützkörpers 6 wird von der Membran 8 hermetisch abgedichtet.

Das Membrangehäuse 10 weist in Anpassung an die Form der Membran 8 entlang der Längsachse L einen zweistufigen Innenquerschnitt auf. Am Außenumfang des breiteren, im montierten Zustand unteren Gehäuseabschnitts ist ein Außengewinde 11 ausgebildet, das der Verschraubung mit einem Innengewinde des Hauptgehäuses 2 dient. Der untere Gehäuseabschnitt des Membrangehäuses 10 wird nach außen von einem Steg 16 flanschartig verbreitert. In dem Innenkantenbereich zwischen dem unteren Gehäuseabschnitt und dem Steg 16 ist der O-Ring 17 aufgenommen, der im konnektierten Zustand (Figur 2) eine Dichtung zwischen dem Membrangehäuses 10 und dem Hauptgehäuse 2 bildet.

Das Membrangehäuse 10 bildet an seiner Oberseite, die im Ausführungsbeispiel frei über das Hauptgehäuse 2 hinausragt, drei erste Konnektierelemente 12. Jedes der Konnektierelemente 12 bildet an der Oberseite seines radial äußeren Randes einen radialen Vorsprung 13 mit einer von der Oberseite aus gesehen rückwärtigen Schulter 14. Die Vorsprünge 13 verjüngen sich je über ihre Schulter 14 nach radial einwärts, so dass von der Oberseite aus gesehen hinter den Vorsprüngen 13 je eine Einschnürung 15 entsteht. Im Längsschnitt des Membrangehäuses 10 bildet jedes der ersten Konnekfierelemente 12 von der Oberseite aus einen sich zunächst nach radial außen wulstartig vorwölbenden und sich anschließend nach radial einwärts zurückwölbenden Vorsprung 13, der sich über die geraden Schultern 14 nach radial einwärts zu der Einschnürung 15 wieder verengt. Jeder der Vorsprünge 13 bildet einen Rastvorsprung und jede der Schultern 14 eine Rastschulter für eine Rastverbindung mit dem Konnektierkopf 22 (Figur 2).

Die ersten Konnektierelemente 12 bilden Rastelemente, die in sich nicht nachgiebig und auch relativ zu dem Membrangehäuse 10 nicht nachgiebig sind. Aufgrund der steifen Verbindung zwischen dem Hauptgehäuse 2 und dem Membrangehäuse 10 sind sie auch relativ zu dem Hauptgehäuse 2 nicht nachgiebig.

In Umfangsrichtung um die Längsachse L gesehen, sind zwischen den Konnektierelementen 12 Aussparungen vorgesehen. Die von den Konnektierelementen 12 gebildeten, aufeinander zu weisenden Seitenwandungen dieser Aussparungen bilden Drehanschläge für ein Werkzeug zum Herstellen und Lösen der Drehverbindung mit dem Hauptgehäuse 2. Die flügelartig von der Längsachse L wegweisenden Konnektierelemente 12 bilden somit in Doppelfunktion eine Montagehilfe. Die Rastvorsprünge 13, die Rastschultern 14 und die Einschnürungen 15 werden von Kreisbögen um die Längsachse L gebildet. Die Aussparungen haben in Bezug auf die Konnektierung keine Funktion. Die Rastvorsprünge 13, Rastschultern 14 und Einschnürungen 15 könnten in Bezug auf die Konnektierfunktion ebenso gut um die Längsachse L je in einem geschlossenen Kreis umlaufen. Die Kreisförmigkeit hat den Vorteil, dass der Konnektierkopf 22 im Rasteingriff der ersten Konnektierelemente 12 und der zweiten Konnektierelemente 24 um die Längsachse L relativ zu dem Membrangehäuse 10 verdreht werden kann. Wegen der Verdrehbarkeit können Scherkräfte zwischen dem Konnektierkopf 22 und dem Perkutanport 1 zu einem großen Teil verhindert werden.

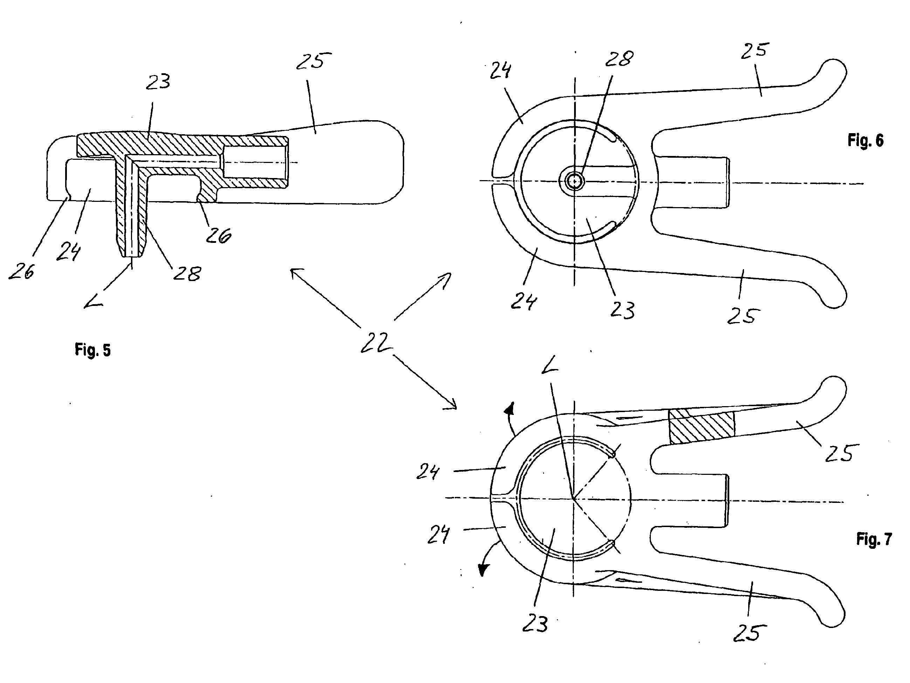

Der Konnektierkopf 22 ist in Figur 5 einzeln und in Figur 2 im konnektierten Zustand je in einem Längsschnitt dargestellt. Ergänzend wird in Bezug auf den Konnektierkopf 22 auch auf die Draufsicht auf seine Unterseite in Figur 6 und die Draufsicht auf seine Oberseite in Figur 7 hingewiesen.

Der Konnektierkopf wird von einem Grundkörper 23, einer Verbindungskanüle 28, den beiden zweiten Konnektierelementen 24 und den beiden Greifelementen 25 gebildet. Der Konnektierkopf 22 ist mit diesen Funktionskomponenten als einstückiger Körper geformt, vorzugsweise als Kunststoffspritzgusskörper. Durch den Grundkörper 23 verläuft von einem rückwärtigen Anschlussbereich bis zur freien Spitze der Verbindungskanüle 28 ein durchgehender Fluidkanal. Im konnektierten Zustand bildet die Längsachse L der Verbindungskanüle 28 auch die Längsachse des Portgehäuses 2, 10. Die Verbindungskanüle 28 und der Mündungsbereich des implantierten oder implantierbaren Fluidführungssystems 5, 6 in dem Perkutanport 1 stehen im konnektierten Zustand somit in einer Flucht. Der von der Verbindungskanüle 28 gebildete Strömungsquerschnitt wird in dem Mündungsbereich des Fluidführungssystems 5, 6 einfach gerade verlängert. Wegen der fluchtenden Anordnung und der Gleichheit der Strömungsquerschnitte wird eine besonders widerstandsarme Strömung erhalten.

Die beiden zweiten Konnektierelemente 24 sind aus einem gemeinsamen Wurzelbereich ausgehend bogenförmig um die Längsachse L bis zu ihren vorderen Enden geführt, zwischen denen ein schmaler, freier Spalt verbleibt. Der Grundkörper 23 bildet den Wurzelbereich bzw. Verbindungsbereich der beiden zweiten Konnektierelemente 24. Jedes der Konnektierelemente 24 ist über diesen Wurzelbereich hinaus rückwärtig zu einem der Greifelemente 25 verlängert. Auf diese Weise wird eine Spreizzange erhalten. Die Konnektierelemente 24 bilden die Zangenbacken und die Greifelemente die Zangenflügel dieser Zange. Wie am besten in den Draufsichten der Figuren 6 und 7 zu erkennen ist, weist der Konnektierkopf 22 eine symmetrische Form in Bezug auf eine Längsebene auf, die die Längsachse L beinhaltet und sich längs durch den rückwärtigen Anschlussbereich und durch den freien Spalt zwischen den vorderen Enden der Konnektierelemente 24 erstreckt.

Die Konnektierelemente 24 bilden an der Unterseite an ihrer Innenmantelfläche eine nach radial einwärts weisende Rastnase 26, die in dem Rasteingriff hinter den Rastvorsprüngen 13 der ersten Konnektierelemente 12 vorschnappt und mit einer Elastizitätskraft gegen die Rastschultern 14 drückt, wie dies in Figur 2 dargestellt ist. Die im Rasteingriff wirkende Elastizitätskraft wird in dem Wurzelbereich des Grundkörpers 23 des Konnektierkopfs 22 erzeugt, nämlich durch eine Spreizung der zweiten Konnektierelemente 24 nach radial auswärts gegen die Rückstellkraft aufgrund der Materialelastizität im Wurzelbereich des Konnektierkopfs 22. Die rückstellende Elastizitätskraft bewirkt, dass die beiden zweiten Konnektierelemente 24 im Rasteingriff mit einer in Bezug auf die Längsachse L nach radial einwärts gerichteten Presskraft gegen die ersten Konnektierelemente 12 drücken. Aufgrund der konischen Form der Rastschultern 14 und/oder einer entsprechenden Gegenform der Rastnase 26 können in dem Rasteingriff zwischen den ersten Konnektierelementen 12 und den zweiten Konnektierelementen 24 beachtliche axiale Zugkräfte übertragen werden, so dass ein unbeabsichtigtes Lösen des Rasteingriffes sicher verhindert werden kann.

Die Rastnase 26 läuft im Ausführungsbeispiel von den aufeinander zu weisenden freien Enden der beiden zweiten Konnektierelemente 24 ausgehend ohne Unterbrechung um nahezu 360° um die Längsachse L. Grundsätzlich ist dies jedoch nicht erforderlich. Allerdings ist die Ausbildung der Rastnase in einem Zug vorteilhaft für die Festigkeit des Rasteingriffs und die freie Verdrehbarkeit des Konnektierkopfs 22 relativ zu dem Perkutanport 1.

Im konnektierten Zustand berührt die Unterseite der zweiten Konnektierelemente 24 eine Oberseite des Stegs 16 des Membrangehäuse 10. Durch den elastischen Presskontakt mit der Rastschulter 14 einerseits und den Kontakt mit der Oberseite des Stegs 16 andererseits, wird ein Befestigungseingriff zwischen den Konnektierelementen 12 und 24 erhalten, der eine Relativbewegung zwischen den Konnektierelementen 12 und 24, und dadurch auch zwischen dem Perkutanport 1 und dem Konnektierkopf 22, in axialer Richtung verhindert. Im Ausführungsbeispiel haben der Perkutanport 1 und der Konnektierkopf 22 Kontakt lediglich über ihre Konnektierelemente 12 und 24. Es wäre jedoch unter Verwendung der gleichen Konnektierelemente 12 und 24 auch möglich, die Unterseite des Grundkörpers 23 unmittelbar auf die Oberseite des Perkutanports 1 zu pressen. Im Ausführungsbeispiel bleibt jedoch zwischen der Unterseite des Grundkörpers 23 und der Oberseite des Perkutanports 1 ein freier Raum, der lediglich von der Verbindungskanüle 28 durchragt wird.

Die Greifelemente 25 sind an den Wurzelbereich des Konnektierkopfs 22 so angeformt, dass eine Bewegung der Greifelemente 25 aufeinander zu in einer radialen Aufspreizbewegung der zweiten Konnektierelemente 24 resultiert. Die Greifelemente 25 sind daher hilfreich für das Lösen des Rasteingriffs.

Die in Figur 1 dargestellte Quetschklammer 30 kann vorteilhafterweise eine Sicherungseinrichtung bilden, indem sie auf dem externen Katheter 21 bis zwischen die beiden Greifelemente 25 geschoben wird und in dieser Position die Greifelemente 25 blockiert, so dass der Rasteingriff nicht unbedachterweise gelöst werden kann.

Im Folgenden wird das Portsystem anhand eines beispielhaften Ablaufs bei einer Implantation im menschlichen Körper beschrieben:
Zunächst wird der im Auslieferungszustand für alle praktischen Anwendungen ausreichend lange Katheter 5 auf eine der speziellen Anwendung gemäße Länge zugeschnitten. Anschließend wird er durch die Gehäuseöffnung der Unterseite des Hauptgehäuses 2 gezogen und vom Operateur implantiert. Der Stützkörper 6 wird in das Hauptgehäuse 2 in die durch das Zentrierelement 7 definierte Position gebracht, und es wird das Membrangehäuse 10 mit der darin eingesetzten Membran 8 in das Hauptgehäuse 2 eingeschraubt, bis es sich gegen den Stützkörper verstemmt und insbesondere dessen Mündung für den implantierten Katheter 5 unter Bildung eines Hohlraums rundherum hermetisch abschließt.

Um den Port 1 unter der Haut zu implantieren, ritzt der Operateur die Haut im Bereich des Ortes der Einführung des Katheters 5 und bildet eine Hauttasche. Der Verankerungskörper 3 wird in die Hauttasche unter die Haut geschoben. In diesem Zustand unmittelbar nach dem Einsetzen befinden sich der Verankerungskörper 3 unter der Haut und das von dem Verankerungskörper 3 aufragende Hauptgehäuse 2 zu einem Teil über der Haut. Das Hauptgehäuse 2 wächst jedoch im Verlaufe der Zeit ein. Um Entzündungen vorzubeugen, ist an der Außenseite des Hauptgehäuses 2 eine Keimbarriere in Form einer im Fußbereich umlaufenden Einschnürung ausgebildet. Diese Keimbarriere soll externen Entzündungskeimen ein Eindringen in das subkutane Gewebes erschweren. Ein Hautstopper in Form eines an dem Hauptgehäuse 2 umlaufenden Vorsprungs schließt die Keimbarriere nach oben ab. Der Hautstopper soll zum einen das Hineinwachsen der Oberhaut begrenzen und zum anderen im Falle einer erforderlichen Wundreinigung ein Eindringen von Fremdkörpern in das subkutane Gewebe verhindern. Nach dem Einwachsen ragt nur noch ein kleinerer, oberer Teil des Hauptgehäuses 2 von außen sichtbar über die Hautoberfläche hinaus hervor. Die Membran 8 dichtet den derart gebildeten Körperzugang nach außen steril ab.

Um Infusionsflüssigkeit über den Perkutanport 1 und den angeschlossenen, implantierten Katheter 5 in den Körper zu führen oder Körperflüssigkeit auf dem umgekehrten Wege zu entnehmen, wird der externe Katheter 21 mit Hilfe des angeschlossenen Katheterkopfs 22 fluidisch und mechanisch mit dem Perkutanport 1 verbunden. Die Verbindung wird auf sehr einfache Weise hergestellt, indem die an der Unterseite über die Konnektierelemente 24 vorstehende Verbindungskanüle 28 in den Durchgang der Membran 8 entlang der Längsachse L eingeführt und bis in die in Figur 2 gezeigte Position hindurchgeführt wird. Durch die eindringende Verbindungskanüle 28 wird die Membran 8 in Ausbuchtungen der Mantelfläche des Membrangehäuses 10 komprimiert. Axial unmittelbar über der Membran 8 ist ein weiterer Ausgleichsraum geschaffen. Die Membran 8 umschließt die Verbindungskanüle 28 während des Eindringens und im konnektierten Zustand allseitig gasdicht.

Im Zuge der Konnektierbewegung, bei der auch die Verbindungskanüle 28 durch die Membran 8 geführt wird, gelangt die Rastnase 26 der Konnektierbacken 24 in Kontakt mit der gerundet abfallenden Oberseite der Rastvorsprünge 13. Durch einen leichten Druck des Konnektierkopfs 22 axial gegen die Oberseite der Rastvorsprünge 13 werden die beiden Konnektierbacken 24 um ihre Spreizachse oder gegebenenfalls auch je um eine eigene Spreizachse elastisch aufgespreizt. Nachdem die Rastnase 26 über die Rastvorsprünge 13 geglitten ist, bewegen sich die beiden Konnektierbacken 24 aufgrund der rückstellenden Elastizitätskraft wieder nach radial einwärts gegen die jeweils zugewandte Rastschulter 14.

Die Konnektierelemente 12 und die Konnektierbacken 24 sind so ausgebildet, dass die Konnektierbacken 24 in dem Rasteingriff nicht vollständig entspannt sind, sondern mit einer verbleibenden Elastizitätsrestkraft gegen die ersten Konnektierelemente 12 drücken. Auf diese Weise wird durch den Rasteingriff eine form- und kraftschlüssige Verbindung zwischen dem Perkutanport 1 und dem Konnektierkopf 22 erhalten. Wegen der Presskraft, die im Rasteingriff zwischen dem Konnektierkopf 22 und dem Portgehäuse 2, 10 über deren Konnektierelemente 12 und 24 wirkt, sind der Konnektierkopf 22 und das Portgehäuse 2, 10 relativ zueinander derart fixiert, dass die Verbindungskanüle 28 relativ zu der Membran 8 durch die in der Praxis zu erwartenden äußeren Zugkräfte und Kippmomente nicht bewegt werden kann. Äußere Druckkräfte werden durch den im Rasteingriff bestehenden Kontakt der zweiten Konnektierelemente 24 mit dem Steg 16 des Portgehäuses 2,10 aufgenommen.

### Bezugszeichen:

- 1: Port
- 2: Hauptgehäuse
- 3: Verankerungskörper
- 4: Öffnungstrichter
- 4a: Führung
- 5: Katheter
- 6: Stützkörper
- 7: Zentrierelement
- 8: Membran
- 9:
- 10: Membrangehäuse
- 11: Gewinde
- 12: Erstes Konnektierelement
- 13: Rastvorsprung
- 14: Rastschulter
- 15: Einschnürung
- 16: Steg
- 17: O-Ring
- 18: -
- 19: -
- 20: Externes Fluidführungssystem
- 21: Katheter
- 22: Konnektierkopf
- 23: Grundkörper
- 24: Zweites Konnektierelement, Konnektierbacke
- 25: Greifelement, Zangenflügel
- 26: Rastnase
- 27: -
- 28: Verbindungskanüle
- 29: -
- 30: Quetschklammer
- L: Längsachse, Konnektierachse

## Patentansprüche

1. Portsystem, umfassend:
a) ein implantierbares erstes Fluidführungssystem (5,6),
b) ein externes, zweites Fluidführungssystem (20) mit einem Konnektierkopf (22) an einem Ende, wobei der Konnektierkopf (22) eine Verbindungskanüle (28) aufweist,
c) einen perkutan implantierbaren Port (1) zur Herstellung einer Fluidverbindung zwischen den Fluidführungssystemen (5,6,20), der ein Portgehäuse (2,10) aufweist, das ein erstes Konnektierelement (12) bildet, wobei in das Portgehäuse (2, 10) ein Anschlussbereich des ersten Führungssystems (5, 6) geführt ist,
d) und eine von dem einstückigen Konnektierkopf (22) gebildete Konnektiervorrichtung (23-26), die ein zweites Konnektierelement (24) umfasst,
e) wobei der Konnektierkopf (22) durch einen lösbaren, form- und kraftschlüssigen Befestigungseingriff der ersten und zweiten Konnektierelemente (12, 24) an dem Portgehäuse (2,10) befestigbar ist,
**dadurch gekennzeichnet, dass**
f) die Verbindungskanüle (28) und der Anschlussbereich des ersten Fluidführungssytems (5, 6) den gleichen Strömungsquerschnit aufweisen.

2. Portsystem nach Anspruch 1 **dadurch gekennzeichnet, dass** die Konnektierelemente (12,24) in dem Befestigungseingriff mit einer Presskraft elastisch aneinander gepresst werden.

3. Portsystem nach dem vorhergehenden Anspruch, **dadurch gekennzeichnet, dass** die Konnektierelemente (12,24) so geformt sind, dass sie in dem Befestigungseingriff mit einer ersten Kraftkomponente parallel zu der Presskraft und einer zweiten Kraftkomponente quer zu der Presskraft gegeneinander drücken.

4. Portsystem nach einem der vorhergehenden Ansprüche, **dadurch gekennzeichnet, dass** die Konnektierelemente (12,24) miteinander in den Befestigungseingriffverrasten.

5. Portsystem nach dem vorhergehenden Anspruch, **dadurch gekennzeichnet, dass** eines der Konnektierelemente (12,24) einen Rastvorsprung (13) mit einer Rastschulter (14) und das andere der Konnektierelemente (12,24) eine Rastnase (26) bildet, die in dem Befestigungseingriff den Rastvorsprung (13) hintergreift und elastisch gegen die Rastschulter (14) drückt.

6. Portsystem nach dem vorhergehenden Anspruch, **dadurch gekennzeichnet, dass** das Konnektierelement (12), das den Rastvorsprung (13) mit der Rastschulter (14) bildet, sich zu einer Einschnürung (15) allmählich verjüngt.

7. Portsystem nach einem der vorhergehenden Anspruch, **dadurch gekennzeichnet, dass** der Konnektierkopf (22) eine Verbindungskanüle (28) aufweist, die in dem Befestigungseingriff der Konnektierelemente (12,24) in das Portgehäuse (2, 10) ragt und durch den Befestigungseingriff von äußeren Kräften befreit oder zumindest entlastet wird.

8. Portsystem nach einem der vorhergehenden Ansprüche, **dadurch gekennzeichnet, dass** der Konnektierkopf (22) einen Grundkörper (23) aufweist und eine mit dem Grundkörper (23) verbundene und von dem Grundkörper (23) abspreizbare Konnektierbacke (24) das zweite Konnektierelement (24) bildet.

9. Portsystem nach einem der vorhergehenden Ansprüche, **dadurch gekennzeichnet, dass** die Konnektiervorrichtung (23-26) eine Zange und eine Backe der Zange das zweite Konnektierelement (24) bilden.

10. Portsystem nach einem der vorhergehenden Ansprüche, **dadurch gekennzeichnet, dass** das erste Konnektierelement (12) nicht nachgiebig an dem Portgehäuse (2,10) angeformt ist.

11. Portsystem nach einem der vorhergehenden Ansprüche, **dadurch gekennzeichnet dass** das Portgehäuse (2,10) ein Hauptgehäuse (2) und ein der Aufnahme einer dichtenden Membran (8) dienendes, in das Hauptgehäuse (2) ragendes und lösbar mit dem Hauptgehäuse (2) verbundenes Membrangehäuse (10) umfasst und dass das erste Konnektierelement (12) von dem Membrangehäuse (10) gebildet wird.

12. Portsystem nach einem der vorhergehenden Ansprüche, **dadurch gekennzeichnet, dass** das erste Konnektierelement (12) an einer dem Konnektierkopf (22) zugewandten Oberseite sich allmählich zu einem Rastvorsprung (13) verbreitert, der um eine Längsachse (L) des hülsenförmigen Portgehäuses (2,10) umläuft, und sich anschließend in Längsrichtung einschnürt, um eine Rastschulter (14) für das zweite Konnektierelement (24) zu bilden.

13. Portsystem nach dem vorhergehenden Anspruch, **dadurch gekennzeichnet, dass** der Rastvorsprung (13) und die Rastschulter (14) in Kurvenbogensegmenten, vorzugsweise in Kreisbogensegmenten, um die Längsachse (L) des Portgehäuses (2, 10) umlaufen.

14. Portsystem nach einem der vorhergehenden Ansprüche, **dadurch gekennzeichnet, dass** das Portgehäuse (2,10) ein Hauptgehäuse (2) und ein der Aufnahme einer Membran (8) dienendes, mit dem Hauptgehäuse (2) drehverbindbares, vorzugsweise verschraubbares, Membrangehäuse (10) umfasst und dass eine Mehrzahl von Flügelelementen, die an einer Oberseite des Portgehäuses (2,10) radial von einer Längsachse (L) des Portgehäuses (2,10) wegweisen und für die Herstellung der Drehverbindung als Drehanschläge für ein Werkzeug dienen, je ein erstes Konnektierelement (12) bilden.

15. Portsystem nach einem der vorhergehenden Ansprüche, **dadurch gekennzeichnet, dass** der Konnektierkopf (22) eine Verbindungskanüle (28) aufweist und dass das erste Fluidführungssystem (5,6) in dem oder in das Portgehäuse (2,10) in einer Flucht der Verbindungskanüle (28) mündet, um Turbulenzen in dem Fluid an einem Übergang zwischen der Verbindungskanüle (28) und dem ersten Fluidführungssystem (5,6) zu vermeiden oder zumindest zu minimieren.

16. Portsystem nach einem der vorhergehenden Ansprüche, **dadurch gekennzeichnet, dass** das Portgehäuse (2,10) an einer Unterseite eine gekrümmte Führung (4a) bildet, um einen an die Führung (4a) angelegten Katheter (5) des ersten Fluidführungssystems (6) knickfrei bis zu einer Gehäuseöffnung umzulenken.

17. Portsystem nach dem vorhergehenden Anspruch, **dadurch gekennzeichnet, dass** das Portgehäuse (2,10) an seiner Unterseite einen Öffnungstrichter (4) bildet, der sich von der Gehäuseöffnung aus erweitert und einen Katheter (5) des ersten Fluidführungssystems (6) gegen Knicken schützt.

18. Portsystem nach Anspruch 1, **dadurch gekennzeichnet, dass** die Konnektiervorrichtung (23-26) an dem Konnektierkopf (22) zangenartig ausgebildet ist und einen Grundkörper (23) des Konnektierkopfes (22) und wenigstens eine Konnektierbacke (24) umfasst, wobei die Konnektierbacke (24) gegen eine rückstellende Elasitzitätskraft von dem Grundkörper (23) abspreizbar ist.

19. Portsystem nach Anspruch 18, **dadurch gekennzeichnet, dass** die Spreizbewegung der wenigstens einen Konnektierbacke (24) um eine Achse (L) ausgeführt wird, die senkrecht zu einer Unterseite des Konnektierkopfs (22) weist.

20. Portsystem nach einem der Ansprüche 18 oder 19, **dadurch gekennzeichnet, dass** die wenigstens eine Konnektierbacke (24) über eine Verbindungsstelle mit dem Grundkörper (23) hinaus durch einen als Greifelement (25) dienenden Zangenflügel verlängert wird.

21. Portsystem nach einem der Ansprüche 18 bis 20, **dadurch gekennzeichnet, dass** die wenigstens eine Konnektierbacke (24) ein den Grundkörper (23) teilweise umgebendes Bogenelement bildet.

22. Portsystem nach einem der Ansprüche 18 bis 21, **dadurch gekennzeichnet, dass** die Konnektiervorrichtung (23-24) eine weitere Konnektierbacke (24) umfasst und die beiden Konnektierbacken (24) gegen die rückstellende Elastizitätskraft voneinander abspreizbar sind.

23. Portsystem nach dem vorhergehenden Anspruch, **dadurch gekennzeichnet, dass** die Konnektierbacken (24) an ihrer Unterseite über einen Kurvenbogen von mehr als 180° um eine zur Unterseite des Konnektierkopfs (22) senkrecht weisende Achse (L) eine nach radial einwärts weisende Rastnase (26) bilden.

24. Portsystem nach einem der vorhergehenden Ansprüche 18 bis 23, **dadurch gekennzeichnet, dass** die wenigstens eine Konnektierbacke (24) materialelastisch mit dem Grundkörper (23) verbunden ist.

25. Portsystem nach einem der Ansprüche 18 bis 24, **dadurch gekennzeichnet, dass** die Konnektierbacke (24) an dem Grundkörper (23) angeformt ist.

26. Portsystem nach einem der Ansprüche 18 bis 25, **dadurch gekennzeichnet, dass** der die Konnektiervorrichtung (23-26) bildende Konnektierkopf (22) einstückig ist.

## Claims

1. A port system, comprising:
a) an implantable first fluid guiding system (5, 6);
b) an external second fluid guiding system (20) comprising a connecting head (22) at one end, wherein the connecting head (22) comprises a connecting cannula (28);
c) a percutaneously implantable port (1) for establishing a fluid connection between the fluid guiding systems (5, 6, 20), the port (1) comprising a port casing (2, 10) which forms a first connecting element (12), wherein a connector region of the first fluid guiding system (5, 6) is guided into the port casing (2, 10);
d) and a connecting device (23-26) which is formed by the one-piece connecting head (22) and comprises a second connecting element (24);
e) wherein the connecting head (22) can be fastened to the port casing (2, 10) by a releasable, positive-fit or force-fit fastening engagement between the first and second connecting elements (12, 24),
**characterised in that**
f) the connecting cannula (28) and the connector region of the first fluid guiding system (5, 6) exhibit the same flow cross-section.

2. The port system according to claim 1, **characterised in that** the connecting elements (12, 24) are elastically pressed onto each other in the fastening engagement with a pressing force.

3. The port system according to the preceding claim, **characterised in that** the connecting elements (12, 24) are moulded such that they press against each other in the fastening engagement, with a first force component parallel to the pressing force and a second force component transverse to the pressing force.

4. The port system according to any one of the preceding claims, **characterised in that** the connecting elements (12, 24) latch together into the fastening engagement.

5. The port system according to the preceding claim, **characterised in that** one of the connecting elements (12, 24) forms a latching protrusion (13) comprising a latching collar (14) and the other of the connecting elements (12, 24) forms a latching projection (26) which, in the fastening engagement, grips behind the latching protrusion (13) and elastically presses against the latching collar (14).

6. The port system according to the preceding claim, **characterised in that** the connecting element (12) which forms the latching protrusion (13) comprising the latching collar (14) gradually tapers towards a constriction (15).

7. The port system according to any one of the preceding claims, **characterised in that** the connecting head (22) comprises a connecting cannula (28) which, in the fastening engagement of the connecting elements (12, 24), protrudes into the port casing (2, 10) and is freed from or at least relieved of external forces by the fastening engagement.

8. The port system according to any one of the preceding claims, **characterised in that** the connecting head (22) comprises a base body (23), and a connecting jowl (24) which is connected to the base body (23) and can be splayed from the base body (23) forms the second connecting element (24).

9. The port system according to any one of the preceding claims, **characterised in that** the connecting device (23-26) forms a pair of pincers and a jowl of the pincers forms the second connecting element (24).

10. The port system according to any one of the preceding claims, **characterised in that** the first connecting element (12) is moulded non-flexibly to the port casing (2, 10).

11. The port system according to any one of the preceding claims, **characterised in that** the port casing (2, 10) comprises a main casing (2) and a membrane casing (10) which serves to accommodate a sealing membrane (8), protrudes into the main casing (2) and is releasably connected to the main casing (2), and **in that** the first connecting element (12) is formed by the membrane casing (10).

12. The port system according to any one of the preceding claims, **characterised in that** the first connecting element (12) gradually flares towards a latching protrusion (13) on an upper side facing the connecting head (22), and the latching protrusion (13) encircles a longitudinal axis (L) of the sleeve-shaped port casing (2, 10) and is then constricted in the longitudinal direction to form a latching collar (14) for the second connecting element (24).

13. The port system according to the preceding claim, **characterised in that** the latching protrusion (13) and the latching collar (14) encircle the longitudinal axis (L) of the port casing (2, 10) in curved arc segments, preferably in circular arc segments.

14. The port system according to any one of the preceding claims, **characterised in that** the port casing (2, 10) comprises a main casing (2) and a membrane casing (10) which serves to accommodate a membrane (8) and can be rotationally connected, preferably screwed, to the main casing (2), and **in that** a plurality of arm elements, which point radially away from a longitudinal axis (L) of the port casing (2, 10) on an upper side of the port casing (2, 10) and serve as rotational stoppers for a tool in order to establish the rotational connection, each form a first connecting element (12).

15. The port system according to any one of the preceding claims, **characterised in that** the connecting head (22) comprises a connecting cannula (28), and **in that** the first fluid guiding system (5, 6) feeds in or into the port casing (2, 10), flush with the connecting cannula (28), in order to avoid or at least minimise turbulence in the fluid at a transition between the connecting cannula (28) and the first fluid guiding system (5, 6).

16. The port system according to any one of the preceding claims, **characterised in that** the port casing (2, 10) forms a curved guide (4a) on an underside, in order to deflect a catheter (5) of the first fluid guiding system (6), which is attached to the guide (4a), to a casing opening without producing kinks.

17. The port system according to the preceding claim, **characterised in that** the port casing (2, 10) forms an opening funnel (4) on its underside, and the opening funnel (4) expands from the casing opening outwards and protects a catheter (5) of the first fluid guiding system (6) from kinks.

18. The port system according to claim 1, **characterised in that** the connecting device (23-26) is formed on the connecting head (22) as a pair of pincers and comprises a base body (23) of the connecting head (22) and at least one connecting jowl (24), wherein the connecting jowl (24) can be splayed from the base body (23) against a restoring elasticity force.

19. The port system according to claim 18, **characterised in that** the splaying movement of the at least one connecting jowl (24) is performed about an axis (L) which points perpendicular to an underside of the connecting head (22).

20. The port system according to any one of claims 18 or 19, **characterised in that** the at least one connecting jowl (24) is extended beyond a connecting point to the base body (23) by a pincer arm serving as a grip element (25).

21. The port system according to any one of claims 18 to 20, **characterised in that** the at least one connecting jowl (24) forms an arc element which partially surrounds the base body (23).

22. The port system according to any one of claims 18 to 21, **characterised in that** the connecting device (23-26) comprises another connecting jowl (24), and the two connecting jowls (24) can be splayed from each other against the restoring elasticity force.

23. The port system according to the preceding claim, **characterised in that** the connecting jowls (24) form a latching projection (26), pointing radially inwards, on their underside via a curved arc of more than 180° about an axis (L) pointing perpendicular to the underside of the connecting head (22).

24. The port system according to any one of claims 18 to 23, **characterised in that** the at least one connecting jowl (24) is connected, materially elastic, to the base body (23).

25. The port system according to any one of claims 18 to 24, **characterised in that** the connecting jowl (24) is moulded to the base body (23).

26. The port system according to any one of claims 18 to 25, **characterised in that** the connecting head (22) forming the connecting device (23-26) is formed in one piece.

## Revendications

1. Système d'accès comprenant :
a) un premier système de guidage de fluide implantable (5,6),
b) un second système de guidage de fluide externe (20) doté d'une tête de raccordement (22) à une extrémité, la tête de raccordement (22) comprenant des canules de raccordement (28),
c) une chambre implantable de façon percutanée (1) destinée à établir une liaison fluidique entre les systèmes de guidage de fluide (5, 6, 20), qui comprend un boîtier de la chambre (2,10), qui forme un premier élément de raccordement (12), une zone de raccordement du premier système de guidage (5, 6) étant introduite dans le boîtier de la chambre (2, 10),
d) et un dispositif de raccordement (23-26) conçu à partir d'une tête de raccordement d'un seul tenant (22), qui comprend un second élément de raccordement (24),
e) la tête de raccordement (22) pouvant être fixée au boîtier de la chambre (2,10) via un engrènement de fixation détachable, à engagement positif et par adhérence des premier et second éléments de raccordement (12, 24),
**caractérisé en ce que**
f) les canules de raccordement (28) et la zone de raccordement du premier système de guidage de fluide (5, 6) comprennent la même section de passage de flux.

2. Système d'accès selon la revendication 1, **caractérisé en ce que** les éléments de raccordement (12,24) sont comprimés les uns sur les autres de manière élastique dans l'engrènement de fixation par une force de compression.

3. Système d'accès selon la revendication précédente, **caractérisé en ce que** les éléments de raccordement (12,24) sont formés de façon à ce qu'ils appuient l'un contre l'autre dans l'engrènement de fixation avec un premier composant de force parallèle à la force de compression et un second composant de force transversale par rapport à la force de compression.

4. Système d'accès selon l'une des revendications précédentes, **caractérisé en ce que** les éléments de raccordement (12,24) s'enclenchent l'un avec l'autre dans l'engrènement de fixation.

5. Système d'accès selon la revendication précédente, **caractérisé en ce qu'**un des éléments de raccordement (12,24) forme une saillie d'enclenchement (13) avec un épaulement d'enclenchement (14) et **en ce que** l'autre élément de raccordement (12,24) forme un ergot d'enclenchement (26), qui vient en prise par derrière avec la saillie d'enclenchement (13) dans l'engrènement de fixation et appuie de façon élastique contre l'épaulement d'enclenchement (14).

6. Système d'accès selon la revendication précédente, **caractérisé en ce que** l'élément de raccordement (12) qui forme la saillie d'enclenchement (13) avec l'épaulement d'enclenchement (14) se réduit graduellement en un étranglement (15).

7. Système d'accès selon l'une des revendications précédentes, **caractérisé en ce que** la tête de raccordement (22) comprend une canule de raccordement (28) qui fait saillie dans l'engrènement de fixation de l'élément de raccordement (12,24) dans le boîtier de la chambre (2, 10) et qui est libérée ou tout au moins déchargée des forces extérieures à travers l'engrènement de fixation.

8. Système d'accès selon l'une des revendications précédentes, **caractérisé en ce que** la tête de raccordement (22) comprend un corps de base (23) et une mâchoire de raccordement (24) pouvant être écartée par le corps de base (23) et reliée au corps de base (23) forme le second élément de raccordement (24).

9. Système d'accès selon l'une des revendications précédentes, **caractérisé en ce que** le dispositif de raccordement (23-26) forme une pince et une mâchoire de la pince forme le second élément de raccordement (24).

10. Système d'accès selon l'une des revendications précédentes, **caractérisé en ce que** le premier élément de raccordement (12) n'est pas formé de manière élastique sur le boîtier de la chambre (2,10).

11. Système d'accès selon l'une des revendications précédentes, **caractérisé en ce que** le boîtier de la chambre (2,10) comprend un boîtier principal (2) et un boîtier à membrane relié de manière détachable au boîtier principal (2) et faisant saillie dans le boîtier principal (2) servant à recevoir une membrane étanche (8)et **en ce que** le premier élément de raccordement (12) est conçu à partir d'un boîtier de membrane (10).

12. Système d'accès selon l'une des revendications précédentes, **caractérisé en ce que** le premier élément de raccordement (12) s'élargit progressivement pour en une saillie d'enclenchement (13) au niveau de la partie supérieure proche de la tête de raccordement (22), qui s'étend autour d'un axe longitudinal (L) du boîtier de la chambre en forme de gaine (2,10) et se rétrécit ensuite dans le sens longitudinal pour former un épaulement d'enclenchement (14) pour le second élément de raccordement (24).

13. Système d'accès selon la revendication précédente, **caractérisé en ce que** la saillie d'enclenchement (13) et l'épaulement d'enclenchement (14) s'étendent autour de l'axe longitudinal (L) du boîtier de la chambre (2, 10) dans des segments courbés, de préférence des segments en arcs de cercle.

14. Système d'accès selon l'une des revendications précédentes, **caractérisé en ce que** le boîtier de la chambre (2,10) comprends un boîtier principal (2) et un boîtier à membrane, pouvant être relié par rotation ou de préférence pouvant être vissé au boîtier principal (2) et servant à recevoir une membrane (8) et **en ce qu'**une pluralité d'éléments d'ailettes, qui sont opposés au niveau de la partie supérieure du boîtier de la chambre (2,10) de façon radiale à l'axe longitudinal (L) du boîtier de la chambre (2,10) et qui servent de butée de rotation pour un outil servant à établir la liaison par rotation, chacun formant un premier élément de raccordement (12).

15. Système d'accès selon l'une des revendications précédentes, **caractérisé en ce que** la tête de raccordement (22) comprend une canule de raccordement (28) et **en ce que** le premier système de guidage de fluide (5,6) débouche dans le boîtier de la chambre (2,10) dans une fuite de la canule de raccordement (28) pour éviter des turbulences dans le fluide au niveau d'un passage entre la canule de raccordement (28) et le premier système de guidage de fluide (5,6) ou tout au moins pour les minimiser.

16. Système d'accès selon l'une des revendications précédentes, **caractérisé en ce que** le boîtier de la chambre (2,10) forme un guidage courbé (4a) au niveau d'une partie inférieure pour faire changer de direction, sans le tordre, un cathéter (5) du premier système de guidage de fluide (6) appuyé contre le guidage (4a) jusqu'à une ouverture du boîtier.

17. Système d'accès selon la revendication précédente, **caractérisé en ce que** le boîtier de la chambre (2,10) forme au niveau de la partie inférieure une ouverture en entonnoir (4) qui s'élargie à partir du boîtier de la chambre et protège un cathéter (5) du premier système de guidage de fluide (6) contre tout flambage.

18. Système d'accès selon la revendication 1, **caractérisé en ce que** le dispositif de raccordement (23-26) est conçu sous forme de pince au niveau de la tête de raccordement (22) et comprend un corps de base (23) de la tête de raccordement (22) et au moins une mâchoire de raccordement (24), la mâchoire de raccordement (24) pouvant être écartée du corps de base (23) contre une force d'élasticité de rappel.

19. Système d'accès selon la revendication 18, **caractérisé en ce que** le mouvement d'expansion de la au moins une mâchoire de raccordement (24) est effectué autour de l'axe (L), qui s'effectue de façon perpendiculaire à la partie inférieure de la tête de raccordement (22).

20. Système d'accès selon l'une des revendications 18 ou 19, **caractérisé en ce que** la au moins une mâchoire de raccordement (24) est allongée via un point de raccordement avec le corps de base (23) par une ailette de pince servant d'élément de préhension (25).

21. Système d'accès selon l'une des revendications 18 à 20, **caractérisé en ce que** la au moins une mâchoire de raccordement (24) forme un élément cintré entourant en partie le corps de base (23).

22. Système d'accès selon l'une des revendications 18 à 21, **caractérisé en ce que** le dispositif de raccordement (23-24) comprend une autre mâchoire de raccordement (24) et les deux mâchoires de raccordement (24) pouvant être écartées l'une de l'autre contre la force d'élasticité de rappel.

23. Système d'accès selon la revendication précédente, **caractérisé en ce que** les mâchoires de raccordement (24) forment sur sa partie inférieure un ergot d'enclenchement (26) tourné vers l'intérieur de façon radiale via une courbe de plus de 180° autour de l'axe (L) tourné de façon perpendiculaire vers la partie inférieure de la tête de raccordements (22).

24. Système d'accès selon l'une des revendications précédentes 18 à 23, **caractérisé en ce que** la au moins une mâchoire de raccordement (24) est reliée de façon élastique au corps de base (23).

25. Système d'accès selon l'une des revendications précédentes 18 à 24, **caractérisé en ce que** la mâchoire de raccordement (24) est formée sur le corps de base (23).

26. Système d'accès selon l'une des revendications précédentes 18 à 25, **caractérisé en ce que** la tête de raccordement (22) formant le dispositif de raccordement (23-26) est conçue d'un seul tenant.
